# EUROPEAN PATENT APPLICATION

(11) **EP 4 692 116 A1**
(43) Date of publication of application: **11.02.2026**
(21) Application number: 24777458.1
(22) Date of filing: 18.01.2024
(51) Int. Cl.: C07K 16/22, C12N 15/13, A61K 39/395, A61P 9/04, A61P 9/10, A61P 9/00, A61P 35/00, A61P 3/04, A61P 3/10, A61P 13/12, G01N 33/68, G01N 33/574

(54) **SINGLE-DOMAIN ANTIBODY OF GDF15 AND USE THEREOF**

(30) Priority: 24.03.2023 CN 202310318320
(71) Applicant: Kexing Biopharm Co., Ltd., Jinan, Shandong 250200 (CN)
(72) Inventor: WEI, Xiling, Jinan, Shandong 250200 (CN); QIN, Suofu, Jinan, Shandong 250200 (CN); HUANG, Huiyu, Jinan, Shandong 250200 (CN); SHANG, Wei, Jinan, Shandong 250200 (CN); SONG, Weihong, Jinan, Shandong 250200 (CN); ZHANG, Hui, Jinan, Shandong 250200 (CN)
(74) Representative: Di Giovine, Paolo
(86) International application number: PCT/CN2024/073056
(87) International publication number: WO 2024/198663

(57) **Abstract**

The present application belongs to the field of biotechnology, and specifically relates to an antibody of GDF15 or the use thereof. Provided is a heavy chain antibody for a GDF15 target. The antibody specifically binds to GDF15 and blocks the interaction between GDF15 and GFRAL, and can be used for treating diseases associated with GDF15 overexpression or diagnosing the level of GDF 15 in a patient. The provided antibody has better blocking activity on the binding of GDF15 to GFRAL than that of the positive control CTL002, better specificity than that of the positive control PF-06946860 and better hydrophilicity than that of the positive control PF-06946860.

## Description

### Cross-reference to related application

The present application claims priority to Chinese Patent Application No. 202310318320.0, and filed on March 24, 2023, the entire disclosure of which is incorporated herein by reference in its entirety.

### Technical Field

The present application relates to the field of biotechnology, specifically, relates to an antibody of GDF15 or use thereof. More precisely, the present application relates to an antibody or its antigen-binding fragment capable of specifically recognizing GDF15, a nucleic acid molecule, an expression vector, a recombinant cell, a pharmaceutical composition, use in pharmaceutical manufacturing, and a kit for detecting GDF15.

### Background Art

Growth differentiation factor 15 (GDF15), also known as macrophage inhibitory cytokine-1 (MIC-1), was identified and discovered by Bootcov et al. in 1997 from an activated macrophage cell line clone. It is a member of the transforming growth factor β (TGFβ) superfamily. GDF15, as a secreted protein circulating in the form of a 25 kDa dimer, consists of two polypeptide chains each containing 112 amino acids, linked together by a disulfide bond. Its receptor is the glial cell line derived neurotrophic factor (GDNF) family receptor α-like (GFRAL) protein. As an α-like receptor of the GDNF family, GFRAL binds to GDF15 and then to the co-receptor RET to form a complex, subsequently activating intracellular signaling pathways consistent with GDNF signaling.

GDF15 and disease treatment: GDF15 is a circulating protein that is expressed in many tissues and organs such as the liver, kidneys, muscles, fat, and placenta under normal physiological conditions, albeit at low levels. However, its expression increases significantly during aging, pregnancy, tissue injury, and various diseases (such as cancer, cardiovascular, and kidney diseases). Many studies have shown (Spanopoulou A et al., Clin Exp Metastasis, 2020, 37: 451-464; Adolph TE et al., J Obes, 2018, 2018: 7108075) that elevated GDF15 levels are associated with cardiovascular diseases such as myocardial hypertrophy, heart failure, atherosclerosis, endothelial dysfunction, as well as obesity, diabetes, cancer, and cachexia. Therefore, blocking or inhibiting the activity of GDF15 (i.e., neutralizing GDF15) can treat the above-mentioned diseases caused by GDF15.

GDF15 and disease diagnosis: GDF15 is elevated in various solid tumor cancer types (Traeger L et al., BMC Cancer. 2019 Jan 15;19(1):74), serving as a diagnostic biomarker for pancreatic cancer, intestinal cancer, ovarian cancer, prostate cancer, primary liver cancer, and lung cancer. It is also associated with disease progression, prognosis, and overall survival in various cancers, including colorectal cancer, gastric cancer, hepatocellular carcinoma, non-small cell lung cancer, urothelial/renal cell carcinoma, ovarian cancer, breast cancer and the like, but not with hematological malignancies. Studies have shown (Zhao D et al., BMC Cancer, 2018, 18: 328.) that serum GDF15 levels in patients with epithelial ovarian cancer resistant to first-line platinum-based chemotherapy (carboplatin or cisplatin) are significantly higher than those in chemotherapy-sensitive patients, and patients with high GDF15 expression have a shorter progression-free survival. In summary, GDF15 can serve as a relatively specific biomarker for cancer diagnosis, prognosis, and chemotherapy resistance. Furthermore, in a two-year follow-up study of patients with acute heart failure (Adela R et al., J Diabetes Res, 2015, 2015: 490842), elevated GDF15 levels were associated with a poorer prognosis in acute heart failure, and GDF15 can also serve as a potential biomarker for assessing the severity and treatment response of cardiovascular diseases.

The work conducted on GDF15 monoclonal antibodies includes: (1) CTL002 (WO2022101263A1), developed by CatalYm, is a humanized monoclonal antibody designed to neutralize GDF-15 produced by tumors, offsetting immune suppression, enhancing the infiltration of immune cells into tumors, improving the priming of dendritic cells on T cells, and enhancing the tumor-killing effects of T cells and NK cells; and (2) PF-06946860 (CN112912395A) developed by Pfizer is a GDF15 monoclonal antibody primarily used in clinical research for anorexia. It aims to improve the anorexia response during chemotherapy in cancer patients through drug intervention, ultimately improving the state of cachexia.

However, only a few types of GDF15 antibodies have been disclose to date, and their specificity still needs to be improved.

### Summary of the Application

The present application is based on the inventor's discovery and understanding of the following facts and problems:
Advantages of heavy chain single-domain antibodies: (1) small molecular weight, strong tissue penetration, widely used in tumor diagnosis and treatment, molecular imaging and the like; (2) simple structure, easy for genetic engineering modification; (3) simple preparation, low production cost and short production cycle for industrial amplification; (4) high stability, easy to store, can resist denaturing conditions such as high temperature, strong acid, strong alkali; and (5) good specificity. Currently, there are few types of publicly available GDF15 antibodies, and there are no reports of heavy chain single-domain antibodies targeting the GDF15 target,

The inventor of the present application performed the following operations: immunizing an alpaca with human GDF15 protein, collecting peripheral blood cells from the immunized alpaca, isolating GDF15-affinity lymphocytes, extracting total RNA, cloning the variable region fragment of the heavy chain of the alpaca heavy-chain antibody using nested PCR technology, inserting the resultant into a phagemid plasmid to construct a phage expression library, then screening the GDF15 antibody through multiple rounds of phage display technology, and finally expressing and purifying the high-affinity antibody obtained through screening in prokaryotic cells, and verifying the binding activity of the obtained single-domain antibody by enzyme-linked immunosorbent assay (ELISA). A GDF15 single-domain antibody with high affinity activity was successfully screened.

The present application provides a heavy-chain antibody targeting the GDF15 target, which specifically binds to GDF15 and blocks the interaction between GDF15 and GFRAL, and can be used to treat diseases associated with GDF15 overexpression or used for the diagnosis of patient GDF15 levels. The antibody provided by the present application exhibits superior blocking activity against the binding of GDF15 to GFRAL compared to the positive control CTL002, superior specificity compared to the positive control PF-06946860, and superior hydrophilicity compared to the positive control PF-06946860.

In the first aspect of the present application, the present application provides an antibody or its antigen-binding fragment capable of specifically recognizing GDF15. According to an embodiment of the present application, the antibody comprises:
(i) an amino acid sequence having the CDR1, CDR2, and CDR3 of a heavy chain variable region as set forth in SEQ ID NOs: 1, 2 and 4-6, wherein the CDR1, CDR2, and CDR3 are determined according to the IMGT definition, Kabat definition, Chothia definition, AbM definition, or Contact definition;
   or, an amino acid sequence with one or more conservative amino acid substitutions compared to (i).

In the second aspect of the present application, the present application provides an antibody or its antigen-binding fragment capable of specifically recognizing GDF15. According to an embodiment of the present application, the antibody comprises:
at least one CDR sequence selected from the following CDR sequences: CDR sequences of the heavy chain variable region: SEQ ID NO: 7, SEQ ID NO: 8, SEQ ID NO: 9, SEQ ID NO: 10, SEQ ID NO: 11, SEQ ID NO: 12, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, and SEQ ID NO: 24, or
an amino acid sequences having at least 90% identity thereto.
YYAIG (SEQ ID NO: 7)
CISSNDGSTYYSDSVKG (SEQ ID NO: 8)
DLTPCPVNPSVDHY (SEQ ID NO: 9)
DYAIG (SEQ ID NO: 10)
CISSSDESTYYADSVKG (SEQ ID NO: 11)
DTECPVQVTSMA (SEQ ID NO: 12)
YYAIG (SEQ ID NO: 16)
CISSSDGSTYYSDSVKG (SEQ ID NO: 17)
GEYGSDCPVQVGS (SEQ ID NO: 18)
AYAVG (SEQ ID NO: 19)
CISSSDGSAYYADSVKG (SEQ ID NO: 20)
DLSCPVQIATFHS (SEQ ID NO: 21)
DLSIG (SEQ ID NO: 22)
CISSSDGSTYYADSVKG (SEQ ID NO: 23)
DREDCPVQPWGIVAGTS (SEQ ID NO: 24)

The antibody according to an embodiment of the present application is capable of specifically binding to GDF15 and inhibiting the activity of GDF 15.

According to an embodiment of the present application, the above-mentioned antibody may further comprise at least one of the following additional technical features:
According to an embodiment of the present application, the antibody comprises:
CDR1, CDR2, and CDR3 sequences of the heavy chain variable region as set forth in the amino acid sequences of SEQ ID NOs: 7, 8 and 9, respectively, or CDR1, CDR2, and CDR3 sequences of the heavy chain variable region having at least 90% identity to the amino acid sequences of SEQ ID NOs: 7, 8 and 9, respectively; or
CDR1, CDR2, and CDR3 sequences of the heavy chain variable region as set forth in the amino acid sequences of SEQ ID NOs: 10, 11 and 12, respectively, or CDR1, CDR2, and CDR3 sequences of the heavy chain variable region having at least 90% identity to the amino acid sequences of SEQ ID NOs: 10 11 and 12, respectively; or
CDR1, CDR2, and CDR3 sequences of the heavy chain variable region as set forth in the amino acid sequences of SEQ ID NOs: 16, 17 and 18, respectively, or CDR1, CDR2, and CDR3 sequences of the heavy chain variable region having at least 90% identity to the amino acid sequences of SEQ ID NOs: 16, 17 and 18, respectively; or
CDR1, CDR2, and CDR3 sequences of the heavy chain variable region as set forth in the amino acid sequences of SEQ ID NOs: 19, 20 and 21, respectively, or CDR1, CDR2, and CDR3 sequences of the heavy chain variable region having at least 90% identity to the amino acid sequences of SEQ ID NOs: 19, 20 and 21, respectively; or
CDR1, CDR2, and CDR3 sequences of the heavy chain variable region CDR1, CDR2, and CDR3 sequences as set forth in the amino acid sequences of SEQ ID NOs: 22, 23, and 24, respectively, or CDR1, CDR2, and CDR3 sequences of the heavy chain variable region having at least 90% identity to the amino acid sequences of SEQ ID NOs: 22, 23, and 24, respectively.

According to an embodiment of the present application, the antibody comprises a heavy chain framework region sequence, and at least a portion of the heavy chain framework region sequence is derived from at least one of a camelid antibody, an alpaca antibody, a murine antibody, a primate antibody, or a mutant thereof.

According to an embodiment of the present application, the heavy chain framework region sequence is derived from an alpaca antibody.

According to an embodiment of the present application, the antibody has a heavy chain variable region with an amino acid sequence as set forth in any one of SEQ ID NOs: 1, 2 and 4-6.

QVQLVESGGGLVQPGGSLRLSCAASGFTLDYYAIGWFRQAPGKGREGVSCISSN
DGSTYYSDSVKGRFTTSRDNAKNTVYLQMNSLKPEDTAVYYCAADLTPCPVNPSVD
HYWGQGTQVTVSS (SEQ ID NO: 1)

QLQLVESGGGLVQPGGSLRLSCVASGFTLDDYAIGWFRQAPGKGLEGVSCISSS
DESTYYADSVKGRFTISRDNAKNTAYLQMNSLKPEDTAIYYCAADTECPVQVTSMA
WGQGTQVTVSS (SEQ ID NO: 2)

QLQLVESGGGLVQPGGSLRLSCAASGFTLDYYAIGWFRQAPGKGPEAVSCISSS
DGSTYYSDSVKGRFTISRDNAKNTVYLQMNSLKPEDTAVYYCAAGEYGSDCPVQV
GSWGQGTQVTVSS (SEQ ID NO: 4)

QVQLVESGGGLVQPGGSLRLACAASGFTLDAYAVGWFRQAPGKNPEGVSCISSS
DGSAYYADSVKGRFTISRDNAKNTVYLQMNSLKPGDTAVYYCAADLSCPVQIATFH
SRGQGTQVTVSS (SEQ ID NO: 5)

QLQLVESGGGEVQPGGSLRLSCAASGATLDDLSIGWFRQAPGKGLEGVSCISSS
DGSTYYADSVKGRFTISRDNAKNTAYLQMNSLRPEDTAVYYCAADREDCPVQPWGI
VAGTSRGQGTQVTVSS (SEQ ID NO: 6)

According to an embodiment of the present application, the antibody contains a heavy chain constant region sequence, and at least a portion of the heavy chain constant region is derived from at least one of a murine antibody, a primate antibody, or a mutant thereof. This further enhances the in vivo half-life of the antibody and improves its stability.

According to an embodiment of the present application, the heavy chain constant region of the antibody is derived from a human IgG antibody or mutant thereof.

According to an embodiment of the present application, the heavy chain constant region of the antibody is derived from human IgG1.

According to an embodiment of the present application, the sequence of Fc in the constant region of the antibody is as set forth in SEQ ID NO: 25.

Amino acid sequence of IgG1 Fc region:

EPKSADKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVS
NKALPAPIEKTISKAKGQPREPQVYTLPPSREEMTKNQVSLTCLVKGFYPSDIAVEWE
SNGQPENNYKTTPPVLDSDGSFFLYSKLTVDKSRWQQGNVFSCSVMHEALHNHYTQ
KSLSLSPGK (SEQ ID NO: 25)

According to an embodiment of the present application, the antibody has a heavy chain with the amino acid sequence as set forth in any one of SEQ ID NOs: 26-30 (full-length of heavy chain single-domain antibody).

Full-length amino acid sequence of LN05:

QVQLVESGGGLVQPGGSLRLSCAASGFTLDYYAIGWFRQAPGKGREGVSCISSN
DGSTYYSDSVKGRFTTSRDNAKNTVYLQMNSLKPEDTAVYYCAADLTPCPVNPSVD
HYWGQGTQVTVSSEPKSADKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSVM
HEALHNHYTQKSLSLSPGK (SEQ ID NO: 26)

Full-length amino acid sequence of LN06:

QLQLVESGGGLVQPGGSLRLSCVASGFTLDDYAIGWFRQAPGKGLEGVSCISSS
DESTYYADSVKGRFTISRDNAKNTAYLQMNSLKPEDTAIYYCAADTECPVQVTSMA
WGQGTQVTVSSEPKSADKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNG
KEYKCKVSNKALPAPIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMHE
ALHNHYTQKSLSLSPGK (SEQ ID NO: 27)

Full-length amino acid sequence of LN13:

QLQLVESGGGLVQPGGSLRLSCAASGFTLDYYAIGWFRQAPGKGPEAVSCISSS
DGSTYYSDSVKGRFTISRDNAKNTVYLQMNSLKPEDTAVYYCAAGEYGSDCPVQV
GSWGQGTQVTVSSEPKSADKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSVM
HEALHNHYTQKSLSLSPGK (SEQ ID NO: 28)

Full-length amino acid sequence of LN14:

QVQLVESGGGLVQPGGSLRLACAASGFTLDAYAVGWFRQAPGKNPEGVSCISSS
DGSAYYADSVKGRFTISRDNAKNTVYLQMNSLKPGDTAVYYCAADLSCPVQIATFH
SRGQGTQVTVSSEPKSADKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGF

YPSDIAVEWESNGQPENNYKTTPPVLDSDGSFFLYSKLTVDKSRWQQGNVFSCSVM
HEALHNHYTQKSLSLSPGK (SEQ ID NO: 29)

Full-length amino acid sequence of LN25:

QLQLVESGGGEVQPGGSLRLSCAASGATLDDLSIGWFRQAPGKGLEGVSCISSS
DGSTYYADSVKGRFTISRDNAKNTAYLQMNSLRPEDTAVYYCAADREDCPVQPWGI
VAGTSRGQGTQVTVSSEPKSADKTHTCPPCPAPELLGGPSVFLFPPKPKDTLMISRTP
EVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCS
VMHEALHNHYTQKSLSLSPGK (SEQ ID NO: 30)

The antibody according to an embodiment of the present application, having a heavy chain with an amino acid sequence as set forth in any one of SEQ ID NOs: 26-30, exhibits high affinity activity towards GDF15, a prolonged half-life in vivo, and enhanced stability.

According to an embodiment of the present application, the antibody is a small molecule antibody.

According to an embodiment of the present application, the small molecule antibody comprises at least one of a single domain antibody, a Fab antibody, a Fv antibody, and a minimal recognition unit.

According to an embodiment of the present application, the antibody is a single-domain antibody.

The present application provides a heavy chain single-domain antibody targeting the GDF15 target. This antibody specifically binds to GDF15 and blocks the interaction between GDF15 and GFRAL, which can be used to treat diseases associated with GDF15 overexpression or used for the diagnosis of patient GDF15 levels. The antibody provided by the present application exhibits superior blocking activity against the binding of GDF15 to GFRAL compared to the positive control CTL002, superior specificity compared to the positive control PF-06946860, and superior hydrophilicity compared to the positive control PF-06946860.

In the third aspect of the present application, the present application provides a nucleic acid molecule. According to an embodiment of the present application, the nucleic acid molecule encodes the antibody or its antigen-binding fragment as described in the first or second aspect.

In the fourth aspect of the present application, the present application provides an expression vector. According to an embodiment of the present application, the expression vector carries the nucleic acid molecule as described in the third aspect.

According to an embodiment of the present application, the expression vector is a prokaryotic expression vector, a eukaryotic expression vector, or a virus.

After introducing the expression vector according to an embodiment of the present application into a host cell, the above-mentioned antibody is expressed under conditions suitable for protein expression, thereby obtaining an antibody with high GDF15 affinity activity.

In the fifth aspect of the present application, the present application provides a recombinant cell. According to an embodiment of the present application, the recombinant cell carries the nucleic acid molecule as described in the third aspect, or the expression vector as described in the fourth aspect, or expresses the antibody or its antigen-binding fragment as described in the first or second aspect.

According to an embodiment of the present application, the recombinant cell is obtained by introducing the expression vector described in the fourth aspect into a host cell. According to an embodiment of the present application, the expression vector is introduced into the host cell by electroporation.

In the sixth aspect of the present application, the present application provides a pharmaceutical composition. According to an embodiment of the present application, the pharmaceutical composition comprises:
the antibody or its antigen-binding fragment as described in the first or second aspect;
the nucleic acid molecule as described in the third aspect;
the expression vector as described in the fourth aspect; or
the recombinant cells as described in the fifth aspect.

The pharmaceutical composition according to an embodiment of the present application can specifically inhibit GDF15, block the binding of GDF15 to GFRAL, and is used for effectively treating or preventing GDF15-related diseases.

In the seventh aspect of the present application, the present application provides the use of the antibody or its antigen-binding fragment as described in the first or second aspect, the nucleic acid molecule as described in the third aspect, the expression vector as described in the fourth aspect, the recombinant cell as described in the fifth aspect, or the pharmaceutical composition as described in the sixth aspect, in the preparation of a drug for diagnosing, treating, or preventing a GDF 15-related disease.

According to an embodiment of the present application, the GDF15-related disease includes a cardiovascular disease, a kidney disease, obesity, diabetes, a tumor, and cachexia.

According to an embodiment of the present application, the cardiovascular disease includes myocardial hypertrophy, heart failure, atherosclerosis, and endothelial dysfunction.

According to an embodiment of the present application, the tumor is a solid tumor, which includes pancreatic cancer, intestinal cancer, ovarian cancer, prostate cancer, primary liver cancer, lung cancer, colorectal cancer, gastric cancer, hepatocellular carcinoma, non-small cell lung cancer, urothelial/renal cell carcinoma, and breast cancer.

In the eighth aspect of the present application, the present application provides a kit for detecting GDF15. According to an embodiment of the present application, the kit comprises the antibody or its antigen-binding fragment according to the first or second aspect.

In the ninth aspect of the present application, the present application provides the use of the antibody or its antigen-binding fragment as described in the first or second aspect, the nucleic acid molecule as described in the third aspect, the expression vector as described in the fourth aspect, and the recombinant cell as described in the fifth aspect, in the preparation of a kit for detecting GDF15 or diagnosing a GDF15-related disease.

In the tenth aspect of the present application, the present application provides a method for diagnosing, treating, or preventing a GDF 15-related disease. According to an embodiment of the present application, the method comprises administering to a subject at least one of the following:
the antibody or its antigen-binding fragment as described in the first or second aspect;
the nucleic acid molecule as described in the third aspect;
the expression vector as described in the fourth aspect;
the recombinant cells as described in the fifth aspect;
the pharmaceutical composition as described in the sixth aspect.

According to the embodiments of the present application, the GDF15-related disease includes a cardiovascular disease, a kidney disease, obesity, diabetes, a tumor, and cachexia.

According to an embodiment of the present application, the cardiovascular disease includes myocardial hypertrophy, heart failure, atherosclerosis, and endothelial dysfunction.

According to the embodiments of the present application, the tumor is a solid tumor, including pancreatic cancer, intestinal cancer, ovarian cancer, prostate cancer, primary liver cancer, lung cancer, colorectal cancer, gastric cancer, hepatocellular carcinoma, non-small cell lung cancer, urothelial/renal cell carcinoma, and breast cancer.

In the eleventh aspect of the present application, the present application provides the use of the antibody or its antigen-binding fragment as described in the first or second aspect, the nucleic acid molecule as described in the third aspect, the expression vector as described in the fourth aspect, the recombinant cell as described in the fifth aspect, or the pharmaceutical composition as described in the sixth aspect in the diagnosis, treatment, or prevention of a GDF15-related disease.

According to the embodiments of the present application, the GDF15-related disease includes a cardiovascular disease, a kidney disease, obesity, diabetes, a tumor, and cachexia.

According to an embodiment of the present application, the cardiovascular disease includes myocardial hypertrophy, heart failure, atherosclerosis, and endothelial dysfunction.

According to an embodiment of the present application, the tumor is a solid tumor, including pancreatic cancer, intestinal cancer, ovarian cancer, prostate cancer, primary liver cancer, lung cancer, colorectal cancer, gastric cancer, hepatocellular carcinoma, non-small cell lung cancer, urothelial/renal cell carcinoma, and breast cancer.

Additional aspects and advantages of the present application will be partially set forth in the following description, and will be partially apparent from the description below, or may be learned through the practice of the present application.

### Brief Description of the Drawings

The above and/or additional aspects and advantages of the present application will become apparent and easily understood from the description of Examples in combination with the accompanying drawings, wherein:
Figure 1 shows the affinity detection results of the GDF15 heavy chain single-domain antibody in Example 3;
Figure 2 shows the detection results of blocking activity of GDF15 heavy chain single-domain antibody at protein level in Example 4;
Figure 3 shows the detection results of blocking activity of GDF15 heavy chain single-domain antibody at cellular level in Example 5;
Figure 4 shows the specificity detection results of GDF15 heavy chain single-domain antibody in Example 6.

### Specific Modes for Carrying Out the Embodiments

The Examples of the present application are described in detail below. The Examples described below are exemplary and are only used to explain the present application, and should not be construed as limiting the present application.

It should be noted that the terms "first" and "second" are used solely for descriptive purposes and should not be construed as indicating or implying relative importance or implicitly specifying the number of technical features indicated. Consequently, features defined with "first" and "second" may explicitly or implicitly include one or more of such features. Furthermore, in the description of the present application, unless otherwise stated, the term "plurality" refers to two or more.

The endpoints and any values disclosed in this text are not limited to the precise ranges or values, and these ranges or values should be understood to include values close to them. For numerical ranges, the endpoint values of each range, between the endpoint values of each range and individual point values, and between individual point values can be combined to obtain one or more new numerical ranges, which should be considered as specifically disclosed in this text.

To facilitate understanding of the present application, certain technical and scientific terms are specifically defined below. Unless otherwise explicitly defined elsewhere in this text, all other technical and scientific terms used in this text have the meanings commonly understood by a person skilled in the art to which the present application pertains.

In this text, the term "comprise" or "include" is used in an open sense, meaning that it includes the content specified in the present application, but does not exclude content from other aspects.

In this text, the terms "optionally", "optional", or "optional" typically refer to events or conditions that may, but not necessarily, occur subsequently, and the description encompasses both scenarios where such events or conditions occur and those where they do not.

### Terminology

The term "antibody" is used in its broadest sense and includes fully assembled antibody, tetrameric antibody, monoclonal antibody, polyclonal antibody, multispecific antibody (e.g., bispecific antibody), antibody fragments (e.g., Fab', F'(ab)2, Fv, single-chain antibody, diabody, Fab) that can bind to antigen, and recombinant peptides comprising the above-mentioned, as long as they exhibit the desired biological activity. "Immunoglobulin" or "tetrameric antibody" refers to a tetrameric glycoprotein composed of two heavy chains and two light chains, each comprising a variable region and a constant region. The antigen-binding portion can be produced through recombinant DNA technology or by enzymatic or chemical cleavage of intact antibodies. Antibody fragments or antigen-binding portions specifically include Fab, Fab', F(ab')2, Fv, domain antibody (dAb), complementarity-determining region (CDR) fragments, CDR-grafted antibody, single-chain antibody (scFv), single-chain antibody fragments, chimeric antibody, diabody, triabody, tetrabody, minibody, linear antibody; chelate recombinant antibody, tribody or bibody, intracellular antibody, nanobody, small modular immunotherapeutic (SMIP), antigen-binding domain immunoglobulin fusion protein, camelized antibody, antibody containing VHH or its variants or derivatives, and polypeptides containing at least a portion of an immunoglobulin which is sufficient to confer antigen-binding specificity to the polypeptide, such as one, two, three, four, five, or six CDR sequences, as long as the antibody retains the required biological activity.

As used in this text, "heavy chain variable region" refers to the region of an antibody molecule that encompasses at least one complementarity determining region (CDR) of the variable domain of the heavy chain of the antibody. The heavy chain variable region may contain one, two, or three CDRs of the heavy chain of the antibody.

In this text, the term "single-domain antibody" refers to a heavy chain antibody naturally found in camel blood/alpaca blood, which lacks the light chain and only includes the heavy chain H-chain with a relatively high molecular weight. The amino acid sequence of the peptide chain at the amino terminal (N-terminal) varies greatly, known as the variable region (V-region), while the carboxyl terminal (C-terminal) is relatively stable with minimal variation, known as the constant region (C-region). The V-region of the H-chain is referred to as VH. Certain regions within the variable region exhibit a higher degree of variation in amino acid composition and sequence, known as the hypervariable region (HVR). The hypervariable region is the site where the antigen binds to the antibody, and thus is also referred to as the complementarity-determining region (CDR). The heavy chain variable region contains three CDR regions. CDR1 and CDR3 are slightly longer than those in humans, and CDR3 protrudes outward in the tertiary structure. Therefore, it is speculated that single-domain antibody has higher specificity and affinity for antigen binding compared to traditional antibody.

The variable domain of a single-domain antibody exhibits a common structure consisting of three hypervariable regions or CDRs connected by relatively conservative framework region (FR). From the N-terminus to the C-terminus, the heavy chain comprises the domains FR1, CDR1, FR2, CDR2, FR3, CDR3, and FR4. The amino acid assignments of each domain conform to the definitions provided in the Kabat Sequences of Proteins of Immunological Interest (National Institutes of Health, Bethesda, Md. (1987 and 1991); or Chothia and Lesk, J. Mol. Biol., 196:901-917, 1987; Chothia et al., Nature, 342:878-883, 1989).

The hypervariable region of an antibody refers to the CDR amino acid residues responsible for antigen binding. The hypervariable region encompasses amino acid residues from the CDRs, such as residues 31-35 (H1), 50-65 (H2), and 95-102 (H3) in the heavy chain variable domain (as described in Kabat et al., "Sequences of Proteins of Immunological Interest," 5th edition, Public Health Service, National Institutes of Health, Bethesda, Maryland (1991)); and/or residues from the hypervariable loops, such as residues 26-32 (H1), 53-55 (H2), and 96-101 (H3) in the heavy chain variable domain (as described in Chothia et al., "J. Mol. Biol." 196:901-917 (1987)).

Monoclonal antibody refers to antibody obtained from a substantially homogeneous population of antibody, in which all antibodies in the mixture have a single amino acid sequence derived from a single clone. Monoclonal antibodies are typically highly specific and target a single antigenic site or epitope. In contrast, polyclonal antibody preparations usually consist of a mixture of antibodies with different amino acid sequences, targeting the same or different determinants (epitopes). In addition to their specificity, monoclonal antibody has the advantage of being synthesized in homogeneous cultures, free from contamination by other immunoglobulins with different specificities and characteristics.

As used in this text, the term "hinge region" refers to the region between the CH1 and CH2 regions of an antibody heavy chain. This region includes the inter-H chain disulfide bond, is rich in proline, does not form α-helices, and is prone to stretching and a certain degree of twisting, which facilitates complementary binding between the antigen-binding site of the antibody and the epitope of the antigen. In the heavy chain single-domain antibody of the present application, the heavy chain variable region is connected to the Fc region through the hinge region.

As used in this text, the term "Fc region" refers to the protein comprising the heavy chain constant region 2 (CH2) and heavy chain constant region 3 (CH3) of immunoglobulin, excluding the heavy chain variable region and heavy chain constant region 1 (CH1). In the present application, the Fc fragment encompasses not only the natural amino acid sequence but also its mutated sequences. The Fc region of immunoglobulin can be derived from humans or animals, such as cattle, goats, pigs, mice, rabbits, hamsters, rats, or guinea pigs.

As used in this text, the term "constant region" refers to the carboxyl terminal (C-terminal) of the polypeptide chain, the 3/4 or 4/5 of the H chain, and the 1/2 segment of the L chain. The number, type, sequence, configuration, and glycosylation content of amino acids in this segment are relatively stable; hence it is called the constant region, also known as the C region. The C regions of the H and L chains are denoted as CH and CL, respectively. The length of CH varies among different classes of immunoglobulins. CH of IgG, IgA, and IgD consists of three segments, including CH1, CH2, and CH3; CH of IgM and IgE consists of four segments, including CH1, CH2, CH3, and CH4. Each heavy chain is composed of a variable region (VH) and the first, second, third, and fourth (optionally) constant regions (CH1, CH2, CH3, and CH4, respectively). Typically, a natural intact antibody takes on a "Y" shape, with the stem of the "Y" structure consisting of the second and third constant regions of the two heavy chains bound via disulfide bonds. Each arm of the "Y" structure includes the VH and CH1 (VH-CH1) of the heavy chain and the light chain (VL-CL).

As used in this text, the term "nucleic acid" is interchangeable with the term "polynucleotide" and refers to deoxyribonucleotides or ribonucleotides and their polymers, in either single-stranded or double-stranded forms, encompassing nucleic acids containing known nucleotide analogs or modified backbone residues or linkages, whether synthetic, naturally occurring, or non-naturally occurring, with binding characteristics similar to those of reference nucleic acids, and metabolized in a manner analogous to reference nucleotides. Examples of such analogs include, but are not limited to, phosphorothioates, phosphonates, methylphosphonates, chiral-methylphosphonates, 2'-O-methylribonucleotides, and peptide-nucleic acids (PNA). Nucleic acids encoding polypeptides or fusion proteins refer to one or more nucleic acid molecules encoding polypeptides or fusion proteins, including such one or more nucleic acid molecules in a single vector or in separate vectors, and such one or more nucleic acid molecules present at one or more locations within a host cell. Unless otherwise stated, a particular nucleic acid sequence also implicitly encompasses its conservatively modified variants (e.g., degenerate codon substitutions) and complementary sequences, as well as sequences explicitly indicated.

As used in this text, the term "vector" refers to a delivery vehicle into which a genetic element (such as the above-mentioned nucleic acid molecule) can be operably inserted and through which the genetic element can be expressed, for example, by producing a protein, RNA, or DNA encoded by the genetic element, or by replicating the genetic element. Vectors can be used to transform, transduce, or transfect host cells, allowing the genetic element they carry to be expressed within the host cells. Examples of vectors include plasmids, phagemids, cosmids, artificial chromosomes such as yeast artificial chromosome (YAC), bacterial artificial chromosome (BAC), or P1-derived artificial chromosome (PAC), phages such as λ phage or M13 phage, and animal viruses. Vectors can contain various expression control elements, including promoter sequences, transcription initiation sequences, enhancer sequences, selection elements, and reporter genes. Additionally, vectors can contain replication initiation sites. Vectors can also include components that assist in their entry into cells, including but not limited to viral particles, liposomes, or protein coatings. Vectors can be expression vectors or cloning vectors. In some embodiments, the vectors provided by the present application (such as expression vectors) contain a nucleic acid sequence encoding a fusion protein according to the present application, at least one promoter (e.g., SV40, CMV, EF-1α) operably linked to the nucleic acid sequence, and at least one selection marker.

As used in this text, the term "host cell" refers to a cell that can be or has been introduced with exogenous polynucleotides and/or vectors. This host cell contains the vector, which can be introduced into mammalian cells to construct the host cell. These host cells can then be used to express the antibody or antigen-binding fragment provided by the present application. By culturing these host cells, the corresponding antibody or fusion protein can be obtained. Available mammalian cells include CHO cells, and the like.

As used in this text, the term "composition" exists in a form that allows the biological activity of the active ingredient to be effective, and does not contain additional ingredients that have unacceptable toxicity to the subject to whom the composition will be administered.

As used in this text, the term "treatment" refers to the temporary or permanent, partial or complete elimination, reduction, inhibition, or amelioration of the clinical symptoms, manifestations, or progression of an event, disease, or condition.

As used in this text, the term "diagnosis" refers to the identification, revelation, ascertainment, and/or localization of the causative process of a pathological state, disease, or condition. In some specific embodiments, the pharmaceutical composition of the present application, when administered to a subject or when exposed to a sample from a subject, aids in the diagnosis of cancer, tumor formation, or related conditions.

In many embodiments, the terms "subject" and "patient" can be used interchangeably, regardless of whether the subject has undergone or is currently undergoing any form of treatment. As used in this text, the term "subject" or "patient" refers to a mammalian subject or patient. Unless otherwise indicated, the terms "patient" or "subject" are used interchangeably throughout this text. Exemplary subjects include, but are not limited to, humans, monkeys, dogs, cats, mice, rats, cows, horses, camels, birds, goats, and sheep. In some embodiments, the subject is a human. In some embodiments, the subject is a human suspected of having cancer, an autoimmune disease or condition, and/or an infection.

### Single-domain antibody

The present application utilizes human GDF15 protein as an antigen to immunize alpacas, obtaining a highly specific and high-affinity anti-GDF15 single-domain antibody (Nanobody, Nb). This antibody can specifically bind to the GDF15 antigen, enabling targeted treatment or prevention of a cardiovascular disease, a kidney disease, obesity, diabetes, a tumor, cachexia, and other diseases.

In some embodiments, the present application provides an antibody or antigen-binding fragment capable of specifically recognizing GDF15, wherein the antibody comprises at least one CDR sequence selected from the following CDR sequences or an amino acid sequence having at least 95% identity thereto: CDR sequences of the heavy chain variable region:
SEQ ID NO: 7, SEQ ID NO: 8, SEQ ID NO: 9, SEQ ID NO: 10, SEQ ID NO: 11, SEQ ID NO: 12, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, and SEQ ID NO: 24.

In other embodiments, the antibody or antigen-binding fragment provided by the present application exhibits conservative amino acid substitutions compared to the above-mentioned heavy chain. "Antigen-binding fragment" refers to an antibody fragment that retains the ability to specifically bind to the antigen (GDF15). "Conservative amino acid substitution" refers to the substitution of an amino acid with another amino acid that is biologically, chemically, or structurally similar. Biologically similar means that the substitution does not disrupt the biological activity of the GDF15 antibody or its interaction with the GDF15 antigen. Structurally similar means that the amino acids have side chains of similar length, such as alanine, glycine, or serine, or have side chains of similar size. Chemical similarity means that the amino acids have the same charge or are both hydrophilic or hydrophobic. For example, hydrophobic residues such as isoleucine, valine, leucine, or methionine can substitute each other. Alternatively, polar amino acids such as arginine can replace lysine, glutamic acid can replace aspartic acid, glutamine can replace asparagine, serine can replace threonine, and so on.

In some embodiments, the present application provides an antibody or antigen-binding fragment, wherein the antibody or antigen-binding fragment comprises CDR1, CDR2, and CDR3 sequences of the heavy chain variable region as set forth in the amino acid sequences of SEQ ID NOs: 7, 8 and 9,, respectively, or CDR1, CDR2, and CDR3 sequences of the heavy chain variable region having at least 90% identity to the amino acid sequences of SEQ ID NO: 7, 8 and 9, respectively; or

CDR1, CDR2, and CDR3 sequences of the heavy chain variable region as set forth in the amino acid sequences of SEQ ID NOs: 10, 11 and 12, respectively, or CDR1, CDR2, and CDR3 sequences of the heavy chain variable region having at least 90% identity to the amino acid sequences of SEQ ID NO: 10 11 and 12, respectively; or

CDR1, CDR2, and CDR3 sequences of the heavy chain variable region as set forth in the amino acid sequences of SEQ ID NOs: 16, 17 and 18, respectively, or having at least 90% identity to the amino acid sequences of SEQ ID NOs: 16, 17 and 18, respectively; or

CDR1, CDR2, and CDR3 sequences of the heavy chain variable region as set forth in the amino acid sequences of SEQ ID NOs: 19, 20 and 21, respectively, or CDR1, CDR2, and CDR3 sequences of the heavy chain variable region having at least 90% identity to the amino acid sequences of SEQ ID NOs: 19, 20 and 21, respectively; or

CDR1, CDR2, and CDR3 sequences of the heavy chain variable region set forth in the amino acid sequences of SEQ ID NOs: 22, 23, and 24, respectively, or CDR1, CDR2, and CDR3 sequences of the heavy chain variable region having at least 90% identity to the amino acid sequences of SEQ ID NOs: 22, 23, and 24, respectively.

In some preferred embodiments, the present application provides an anti-GDF15 single-domain antibody, which comprises a heavy chain variable region with the amino acid sequence as set forth in any one of SEQ ID NOs: 1, 2 and 4-6, or a heavy chain with the amino acid sequence as set forth in any one of SEQ ID NOs: 26-30.

The antibody against GDF15 in the present application is a variable domain of heavy chain of heavy-chain (VHH), commonly known as a "nanobody". The inventor immunized an alpaca with GDF15 recombinant protein, constructed and screened a phage library, and obtained a GDF15 heavy chain single-domain antibody. The GDF15 heavy chain single-domain antibody in the present application can be obtained by constructing expression vectors using the GDF15 monoclonal antibody sequences, transiently transfecting CHO cells, and purifying using affinity chromatography with ProteinA packing.

The inventor evaluated the affinity of the obtained GDF15 monoclonal antibody protein using the ELISA method; assessed the blocking activity of the GDF15 heavy chain single-domain antibody against GDF15 using the GDF15-GFRAL ligand-receptor ELISA binding assay; evaluated the blocking activity of the GDF15 heavy chain single-domain antibody against GDF15 using the reporter gene method; detected the binding of the GDF15 heavy chain single-domain antibody to GDF15 homolog proteins GDNF, ARTN, and NRTN using the ELISA method to investigate whether the GDF15 heavy chain single-domain antibody would bind to GDF15 homolog proteins; and detected the hydrophilicity and hydrophobicity of the GDF15 heavy chain single-domain antibody using the hydrophobic interaction chromatography (HIC) method. The results showed that the novel GDF15 heavy chain single-domain antibodies provided by the present application specifically bind to GDF15 and block the interaction between GDF15 and GFRAL, which can be used for treating diseases associated with GDF15 overexpression or for diagnosing patient GDF15 levels.

### Nucleic acid molecules, expression vectors, recombinant cells

During the preparation or acquisition of these antibodies, nucleic acid molecules expressing these antibodies can be utilized to link with different vectors, and then expressed in different cells to obtain the corresponding antibodies.

The present application further provides an isolated nucleic acid molecule encoding the above-mentioned antibody or antigen-binding fragment.

The present application also provides an expression vector comprising the isolated nucleic acid molecule mentioned above. When connecting the isolated polynucleotide to the vector, the polynucleotide can be directly or indirectly linked to the control elements on the vector, as long as these control elements are capable of controlling the translation and expression of the polynucleotide. Of course, these control elements can either be directly derived from the vector itself or exogenous, meaning they are not derived from the vector itself. Naturally, the polynucleotide and the control elements should be operably linked. In this context, "operably linked" refers to connecting the exogenous gene to the vector so that the control elements within the vector, such as transcriptional control sequences and translational control sequences, can exert their expected function of regulating the transcription and translation of the exogenous gene. Of course, the polynucleotides encoding antibody heavy chains can be inserted independently into different vectors, although they are commonly inserted into the same vector. Commonly used vectors include plasmids, phages, and so on. For example, the pcDNA3.4 plasmid can be used.

The present application also provides a recombinant cell containing the expression vector. The expression vector can be introduced into prokaryotic cells to construct recombinant cells, which can then be used to express the antibodies or antigen-binding fragments provided by the present application. By culturing these recombinant cells, the corresponding antibodies can be obtained.

Pharmaceutical compositions, kits, and uses in pharmaceutical manufacturing, as well as uses in the preparation of kits.

The present application further provides a pharmaceutical composition comprising the above-mentioned antibody or antigen-binding fragment, and a pharmaceutically acceptable carrier.

The anti-GDF15 antibody provided in this text can be incorporated into pharmaceutical compositions suitable for administration to subjects. Typically, these pharmaceutical compositions include the anti-GDF15 antibody provided in this text and a pharmaceutically acceptable carrier. "Pharmaceutically acceptable carriers" can include any and all solvents, dispersion media, coatings, antibacterial and antifungal agents, isotonic agents, and delayed absorption agents that are physiologically compatible. Specific examples can be one or more of water, saline, phosphate-buffered saline, glucose, glycerol, ethanol, and their combinations. In many cases, isotonic agents such as sugars, polyols (such as mannitol, sorbitol), or sodium chloride are included in the pharmaceutical compositions. Of course, pharmaceutically acceptable carriers can also include trace amounts of auxiliary substances, such as wetting agents or emulsifiers, preservatives, or buffers, to extend the shelf life or efficacy of the antibody.

For example, the antibody of the present application can be incorporated into pharmaceutical compositions suitable for parenteral administration (such as intravenous, subcutaneous, intraperitoneal, and intramuscular). These pharmaceutical compositions can be prepared in various forms, such as liquid, semi-solid, and solid dosage forms, including but not limited to liquid solutions (such as injection solutions and infusion solutions), dispersants or suspensions, tablets, pills, powders, liposomes, and suppositories. Typical pharmaceutical compositions are in the form of injection solutions or infusion solutions. The antibody can be administered via intravenous infusion or injection, or intramuscular or subcutaneous injection.

Of course, the anti-GDF15 antibody mentioned in this text can also be made into a kit or a portion of other diagnostic reagents as needed. According to an embodiment of the present application, the present application also provides a kit, which includes the above-mentioned GDF15 antibody. The kit provided by the present application can be used, for example, for immunoblotting, immunoprecipitation, and other kits involving the use of the specific binding properties of GDF15 antigen and antibody for detection. These kits may contain any one or more of the following: antagonist, anti-GDF15 antibody, or drug reference material; protein purification column; immunoglobulin affinity purification buffer; cell assay diluent; instructions or literature and the like. The anti-GDF15 antibody can be used in different types of diagnostic tests, such as detecting the presence of various diseases or drugs, toxins, or other proteins in vitro or in vivo. For example, the serum or blood of the subject can be tested to diagnose related diseases. Such related diseases may include GDF15-related diseases, such as cardiovascular disease, kidney disease, obesity, diabetes, tumors, cachexia and the like.

When utilizing the anti-GDF15 antibody provided by the present application to treat the above-mentioned diseases, it suffices to administer the anti-GDF15 antibody to the subject. The present application provides a method for treating the above-mentioned diseases, which comprises administering the antibody or its antigen-binding fragment provided by the present application to a subject in need.

The following will explain the scheme of this disclosure in combination with Examples. A person skilled in the art will understand that the following Examples are only used to illustrate this disclosure and should not be considered as limiting the scope of this disclosure. If specific technologies or conditions are not specified in the Examples, they shall be in accordance with the technologies or conditions described in the literature within the field or in accordance with the product specifications. If the manufacturer of the reagents or instruments used is not specified, they are all conventional products that can be purchased on the market.

### Example 1: Screening of heavy chain single-domain antibodies targeting GDF15

### 1. Construction of a phage library

Two alpacas were selected, and immunized five times with human GDF15 protein (Novoprotein, Cat# DRA26), after the fifth immunization, 50 mL of peripheral blood was collected from each alpaca, lymphocytes were isolated using lymphocyte separation medium (GE, Cat# 17-1440-03), total RNA was extracted using RNA extraction reagent (RNAiso Plus), the extracted RNA was reverse transcribed into cDNA using the PrimeScript II kit (TAKARA, Cat# 6210A). Then, the VHH target gene fragment, namely the variable region fragment of heavy chain antibody, was amplified using nested PCR.

After digesting the target gene VHH and vector pComb3xss with SfiI, and linking them using T4 DNA ligase, the resultant was transformed into TG1 electrocompetent cells to construct a VHH gene library. Simultaneously, 0.1 µL, 0.01 µL, 0.001 µL, and 0.0001 µL of the mixed transformation solution were evenly plated onto 90 mm petri dishes, resulting in a calculated library capacity of 2.2×10⁹ cfu. 100 colonies were randomly selected for amplification and sequencing; the results indicated an insertion efficiency of 100%.

A volume of living cells representing 10-100 times the capacity of the gene library was taken for inoculation and culture. After culturing to the logarithmic phase, M13K07 phage was used for rescue. After rescue culture, the phage was centrifuged and collected, the phage was purified using PEG-NaCl, and the phage display library with a titer of 2.0×10¹³ cfu/mL was obtained. It can be directly used for subsequent affinity screening of specific phages.

### 2. Phage library screening

The plate was coated with 100 µg/mL and 50 µg/mL of human GDF15 protein (Novoprotein, Cat# DRA26), and incubated overnight at 4°C. On the next day, it was blocked with 1% OVA at 37°C for 1 hour. 100 µL of phages (2.0×10¹³ cfu/mL) was added and incubated. Then, it was washed five times with PBST (PBS containing 0.05% Tween 20) to remove unbound phages. Finally, the phages specifically bound to human GDF15 protein was eluted with 100 µL of Gly-HCl (pH2.2), and used to infect E. coli TG1 in the logarithmic growth phase to produce and purify phages for the next round of screening. After repeating the same screening process twice, the obtained phages were used to infect E. coli TG1 and plated. Monoclonal colonies were picked from the plate for sequencing. The protein sequences of each clone were analyzed based on sequence alignment results, and clones with different CDR1, CDR2, and CDR3 sequences were regarded as different antibody strains. A total of six different antibodies were obtained, and the antibody sequences are shown in Table 1.

**Table 1. Amino acid sequence of the variable region of GDF15 heavy chain single-domain antibody (Numbering Scheme Kabat)**

| Clone number | Sequence | SEQ ID NO: |
|---|---|---|
| LN05 | QVQLVESGGGLVQPGGSLRLSCAASGFTLDYYAIGWFRQAPG KGREGVSCISSNDGSTYYSDSVKGRFTTSRDNAKNTVYLQM NSLKPEDTAVYYCAADLTPCPVNPSVDHYWGQGTQVTVSS | 1 |
| | CDR1: YYAIG (SEQ ID NO: 7) | 7 |
| | CDR2: CISSNDGSTYYSDSVKG (SEQ ID NO: 8) | 8 |
| | CDR3: DLTPCPVNPSVDHY (SEQ ID NO: 9) | 9 |
| LN06 | QLQLVESGGGLVQPGGSLRLSCVASGFTLDDYAIGWFRQ APGKGLEGVSCISSSDESTYYADSVKGRFTISRDNAKNT AYLQMNSLKPEDTAIYYCAADTECPVQVTSMAWGQGT QVTVSS | 2 |
| | CDR1: DYAIG (SEQ ID NO: 10) | 10 |
| | CDR2: CISSSDESTYYADSVKG (SEQ ID NO: 11) | 11 |
| | CDR3: DTECPVQVTSMA (SEQ ID NO: 12) | 12 |
| LN11 | QVQLVESGGGLVQPGGSLRLSCAASGFTIRTYTMSWYR QAPGKERALVAYITPGGPTYHIDSVKGRFTISRNNAENT VYLQMNSLKPEDTAVYYCGNGFGSWGQGTQVTVSS | 3 |
| | CDR1: TYTMS (SEQ ID NO: 13) | 13 |
| | CDR2: YITPGGPTYHIDSVKG (SEQ ID NO: 14) | 14 |
| | CDR3: GFGS (SEQ ID NO: 15) | 15 |
| LN13 | QLQLVESGGGLVQPGGSLRLSCAASGFTLDYYAIGWFR QAPGKGPEAVSCISSSDGSTYYSDSVKGRFTISRDNAK NTVYLQMNSLKPEDTAVYYCAAGEYGSDCPVQVGSW GQGTQVTVSS | 4 |
| | CDR1: YYAIG (SEQ ID NO: 16) | 16 |
| | CDR2: CISSSDGSTYYSDSVKG (SEQ ID NO: 17) | 17 |
| | CDR3: GEYGSDCPVQVGS (SEQ ID NO: 18) | 18 |
| LN14 | QVQLVESGGGLVQPGGSLRLACAASGFTLDAYAVGWFRQ APGKNPEGVSCISSSDGSAYYADSVKGRFTISRDNAKNTV YLQMNSLKPGDTAVYYCAADLSCPVQIATFHSRGQGTQV TVSS | 5 |
| | CDR1: AYAVG (SEQ ID NO: 19) | 19 |
| | CDR2: CISSSDGSAYYADSVKG (SEQ ID NO: 20) | 20 |
| | CDR3: DLSCPVQIATFHS (SEQ ID NO: 21) | 21 |
| LN25 | QLQLVESGGGEVQPGGSLRLSCAASGATLDDLSIGWFRQAP GKGLEGVSCISSSDGSTYYADSVKGRFTISRDNAKNTAYLQ MNSLRPEDTAVYYCAADREDCPVQPWGIVAGTSRGQGTQ VTVSS | 6 |
| | CDR1: DLSIG (SEQ ID NO: 22) | 22 |
| | CDR2: CISSSDGSTYYADSVKG (SEQ ID NO: 23) | 23 |
| | CDR3: DREDCPVQPWGIVAGTS (SEQ ID NO: 24) | 24 |

### Example 2: Preparation of GDF15 heavy chain single-domain antibody

The variable region sequence of GDF 15 heavy chain single-domain antibody (see Table 1) was connected to the human IgG1 Fc sequence, synthesized into the expression plasmid pcDNA3.4, and transformed into E. coli, and then the plasmid was extracted. The expression plasmid was filtered through a 0.22 µm filter, mixed with CHO cells and electroporation buffer, and the mixture was added to a 1 mL electroporation cuvette. After electroporation, the cells in the electroporation cuvette were transferred to a shake flask prefilled with 20ml of culture medium, and incubated statically for 40 minutes. After incubation, the shake flask was placed in a 37°C, 270 rpm, and 8% CO₂ environment for culture. After 24 hours, feed supplement/sodium butyrate/penicillin-streptomycin (Gibco, Cat# 15140-122) was added, and the culture was continued for 3-6 days. Then, the cell supernatant was collected. The cell supernatant was loaded onto a ProteinA affinity chromatography column, and the chromatography column was washed with 20 mL of PBS buffer. Subsequently, 5 mL of sodium acetate buffer (pH3.4) was added to elute the chromatography column, and the eluate was collected in a dialysis bag and dialyzed against PBS buffer to obtain the target product, the GDF15 heavy chain single-domain antibody. Table 2 below shows the Fc region sequence of the GDF15 heavy chain single-domain antibody and the full-length sequence of each antibody.

**Table 2: Amino acid sequence of the Fc region and full-length sequence of the GDF15 heavy chain single-domain antibody**

| Name | Sequence | SEQ ID NO: |
|---|---|---|
| IgG1 Fc region | EPKSADKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLT VDKSRWQQGNVFSCSVMHEALHNHYTQKSLSLSPGK | 25 |
| LN05 full-length | QVQLVESGGGLVQPGGSLRLSCAASGFTLDYYAIGWFRQAPGKGREGV SCISSNDGSTYYSDSVKGRFTTSRDNAKNTVYLQMNSLKPEDTAVYYCA ADLTPCPVNPSVDHYWGQGTQVTVSSEPKSADKTHTCPPCPAPELLGGPS VFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISK AKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYT QKSLSLSPGK | 26 |
| LN06 full-length | QLQLVESGGGLVQPGGSLRLSCVASGFTLDDYAIGWFRQAPGKGLEGVSC ISSSDESTYYADSVKGRFTISRDNAKNTAYLQMNSLKPEDTAIYYCAADTE CPVQVTSMAWGQGTQVTVSSEPKSADKTHTCPPCPAPELLGGPSVFLFPPK PKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQ VYTLPPSREEMTKNQVSLTCLVKGFYPSDIAVEWESNGQPENNYKTTPPVLD SDGSFFLYSKLTVDKSRWQQGNVFSCSVMHEALHNHYTQKSLSLSPGK | 27 |
| LN13 full-length | QLQLVESGGGLVQPGGSLRLSCAASGFTLDYYAIGWFRQAPGKGPEAVSCISS SDGSTYYSDSVKGRFTISRDNAKNTVYLQMNSLKPEDTAVYYCAAGEYGSDC PVQVGSWGQGTQVTVSSEPKSADKTHTCPPCPAPELLGGPSVFLFPPKPKDTL MISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPSR EEMTKNQVSLTCLVKGFYPSDIAVEWESNGQPENNYKTTPPVLDSDGSFFLY SKLTVDKSRWQQGNVFSCSVMHEALHNHYTQKSLSLSPGK | 28 |
| LN14 full- | QVQLVESGGGLVQPGGSLRLACAASGFTLDAYAVGWFRQAPGKNPEGVSC ISSSDGSAYYADSVKGRFTISRDNAKNTVYLQMNSLKPGDTAVYYCAADLS | 29 |
| length | CPVQIATFHSRGQGTQVTVSSEPKSADKTHTCPPCPAPELLGGPSVFLFPPKP KDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQV YTLPPSREEMTKNQVSLTCLVKGFYPSDIAVEWESNGQPENNYKTTPPVLDS DGSFFLYSKLTVDKSRWQQGNVFSCSVMHEALHNHYTQKSLSLSPGK | |
| LN25 full-length | QLQLVESGGGEVQPGGSLRLSCAASGATLDDLSIGWFRQAPGKGLEGV SCISSSDGSTYYADSVKGRFTISRDNAKNTAYLQMNSLRPEDTAVYYCA ADREDCPVQPWGIVAGTSRGQGTQVTVSSEPKSADKTHTCPPCPAPELLG GPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKT ISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHY TQKSLSLSPGK | 30 |

### Example 3: Affinity detection of GDF15 heavy chain single-domain antibody

The human GDF15 protein (Novoprotein, Cat# DRA26) was diluted to prepare a 1µg/ mL coating solution, and 100 µL per well of this solution was added to the enzyme-linked immunosorbent assay plate and the plate was incubated at 2~8°C for 12 hours or more for coating. After the residual coating solution was discarded, 2% BSA-PBS was added at 200 µL per well, and the plate was incubated at 37°C for 2 hours for blocking. Then, the plate was washed three times with 300 µL of PBST per well. The test samples (LN05, LN06, LN11, LN13, LN14, LN25, PF-06946860, and CTL002) were diluted to 20 nM with PBS and further serially diluted 3-fold for 8 gradients. Each dilution was added to the plate at 100 µL per well and the plate was incubated at 37°C for 1 hour. Then, the plate was washed three times with 300 µL of PBST per well, and then Goat anti-Human Fc-HRP (abcam, Cat# ab97225), diluted 10,000-fold, was added at 100 µL per well and the plate was incubated at 37 °C for 1 hour. Then, the plate was washed three times with 300 µL of PBST per well, and tapped dry. TMB chromogenic solution was added at 100 µL per well and allowed to react at room temperature for 5 minutes. The reaction was stopped by adding 50 µL of 2 M H₂SO₄ per well. The plate with the reaction stopped was placed on the microplate reader, and the OD450 absorbance value was measured at a wavelength of 450 nm using a microplate reader. The data were analyzed using GraphPad Prism 9.0 software to fit the binding activity and obtain the EC50 value of GDF 15 binding activity.

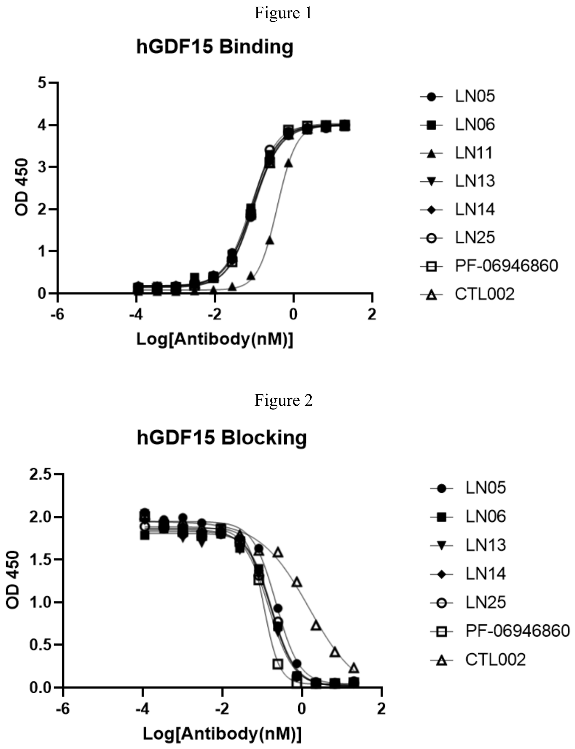

The smaller the EC50 value, the stronger the binding activity. The results are shown in Table 3 and Figure 1, and they indicate that LN05, LN06, LN13, LN14, and LN25 exhibit significant binding activity to human GDF15, comparable to that of PF-06946860 (Pfizer) and CTL002 (CatalYM). LN11 also exhibits significant binding activity to human GDF15, but binding activity is weaker than that of PF-06946860 and CTL002.

**Table 3. Affinity of GDF15 heavy chain single-domain antibody**

| Sample | EC50(nM) |
|---|---|
| LN05 | 0.09125 |
| LN06 | 0.08322 |
| LN11 | 0.38180 |
| LN13 | 0.08585 |
| LN14 | 0.09879 |
| LN25 | 0.08918 |
| PF-06946860 | 0.09738 |
| CTL002 | 0.09328 |

### Example 4: Study on the blocking activity of GDF15 heavy chain single-domain antibody at protein level

The GFRAL protein (KACTUS, Cat# GFL-HM401) was diluted to prepare a 1 µg/mL coating solution, and added to an enzyme-linked immunosorbent assay plate at 100 µL/well, and the plate was incubated at 2~8°C for 12 hours or more for coating. After the residual coating solution was discarded, 2% BSA-PBS was added at 200 µL per well, and the plate was incubated at 37°C for 2 hours for blocking. Then, the plate was washed three times with 300 µL of PBST per well. A sample dilution solution containing 20 ng/mL GDF15-Biotin protein (KACTUS, Cat# GDF-HM215B) was prepared and used to dilute the test samples (LN05, LN06, LN13, LN14, LN25, PF-06946860, and CTL002) to 20nM, followed by serial 3-fold dilutions for 12 gradients. Each dilution was added to the plate at 100 µL per well and the plate was incubated at 37°C for 1 hour. Then, the plate was washed three times with 300 µL of PBST per well, and then Streptavidin (HRP) (abcam, Cat# ab7403) diluted 30,000-fold was added at 100 µL per well and incubated at 37 °C for 1 hour. Then, the plate was washed three times with 300 µL of PBST per well, and tapped dry. TMB chromogenic solution was added at 100 µL per well and allowed to react at room temperature for 5 minutes. The reaction was stopped by adding 50 µL of 2 M H₂SO₄ per well. The plate with the reaction stopped was placed on the microplate reader, and the OD450 absorbance value was measured at a wavelength of 450 nm using a microplate reader. The data were analyzed using GraphPad Prism 9.0 software to fit the inhibitory activity and obtain the ELISA blocking activity IC50 value.

The smaller the IC50 value, the stronger the blocking activity. The results are shown in Table 4 and Figure 2, and indicate that LN05, LN06, LN13, LN14, and LN25 exhibit significant dose-dependent blocking activity against the binding of human GDF15 to its receptor GFRAL, and the blocking activity is comparable to that of PF-06946860 and superior to that of CTL002.

**Table 4. ELISA blocking activity IC50 of GDF15 heavy chain single-domain antibody**

| Sample | IC50(nM) |
|---|---|
| LN05 | 0.2213 |
| LN06 | 0.1747 |
| LN13 | 0.1474 |
| LN14 | 0.1604 |
| LN25 | 0.1713 |
| PF-06946860 | 0.1097 |
| CTL002 | 1.5010 |

### Example 5: Study on the blocking activity of GDF15 heavy chain single-domain antibody at the cellular level

H-GDF15 Reporter 293 cell suspension (Genomeditech, Cat# GM-C06718) was added to a 96-well microplate (BeyoGold, Cat# FCP968) at a density of 1.3*10⁴ cells/50 µL/well, and incubated overnight in a carbon dioxide incubator. The microplate was taken out and added with 50 µL of a mixture culture medium containing GDF15 (final concentration of 5ng/mL, Novoprotein, Cat# DRA26) and serially diluted test samples (LN05, LN06, LN13, LN14, LN25, PF-06946860, CTL002) (starting working concentration: 5.13 nM, 3-fold dilutions, 9 concentrations in total) was added to each well, and the plate was returned to the 37°C, 5 % CO₂ incubator for continuous incubation. After 17 hours, 100 µL of luciferase reporter gene detection reagent (Vazyme, Cat# DD1203) was added to each well, incubated for 15 minutes, and the chemiluminescence value RLU was readed. The data were analyzed using GraphPad Prism 8.0 software to fit the inhibition activity and obtain the IC50 value of GDF15 blocking activity (GDF15 heavy chain single-domain antibody blocks the binding of GDF15 to GFRAL).

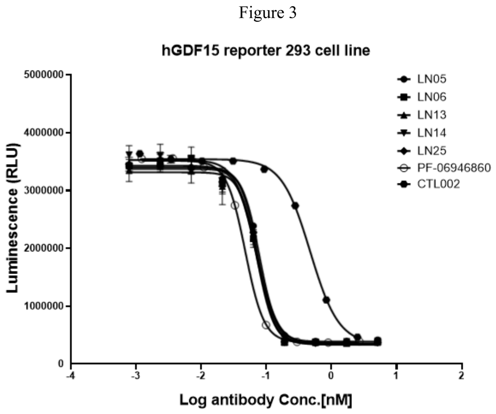

The smaller the IC50 value, the stronger the blocking activity. The results are shown in Table 5 and Figure 3, and they indicate that LN05, LN06, LN13, LN14, and LN25 exhibit significant dose-dependent blocking activity against GDF15-mediated reporter gene expression, and the blocking activity is comparable to that of PF-06946860, and superior to that of CTL002.

**Table 5. IC50 of GDF15 reporter gene activity**

| Sample | IC50 (nM) |
|---|---|
| LN05 | 0.07778 |
| LN06 | 0.07052 |
| LN13 | 0.07190 |
| LN14 | 0.07009 |
| LN25 | 0.07347 |
| PF-06946860 | 0.04751 |
| CTL002 | 1.99000 |

### Example 6: Specificity study of GDF15 heavy chain single-domain antibody

Human GDNF protein (Novoprotein, Cat# C226), ARTN protein (Novoprotein, Cat# CR34), and NRTN protein (Novoprotein, Cat# CE27) were diluted to prepare 1 µg/mL coating solutions, and each solution was added to an ELISA plate at 100 µL/well, and the plate was incubated at 2~8°C for 12 hours or more for coating.

After the residual coating solution was discarded, 2% BSA-PBS was added at 200 µL per well, and the plate was incubated at 37°C for 2 hours for blocking. Then, the plate was washed three times with 300 µL of PBST per well. The test samples (LN05, LN06, LN13, LN14, LN25, and PF-06946860) were diluted to 2,000 nM with PBS and further serially diluted 3-fold for 12 gradients. Each dilution was added to the plate at 100 µL per well and incubated at 37°C for 1 hour. Then, the plate was washed three times with 300 µL of PBST per well, and then Goat anti-Human Fc-HRP (abcam, Cat# ab97225) diluted 10,000-fold was added at 100 µL per well and incubated at 37 °C for 1 hour. Then, the plate was washed three times with 300 µL of PBST per well, and tapped dry. TMB chromogenic solution was added at 100 µL per well and allowed to react at room temperature for 5 minutes. The reaction was stopped by adding 50 µL of 2 M H₂SO₄ per well. The plate with the reaction stopped was placed on the microplate reader, and the OD450 absorbance value was measured at a wavelength of 450 nm using a microplate reader. The data were analyzed using GraphPad Prism 9.0 software to fit the binding activity and obtain the EC50 value of GFRA1, GFRA2, and GFRA3 binding activity.

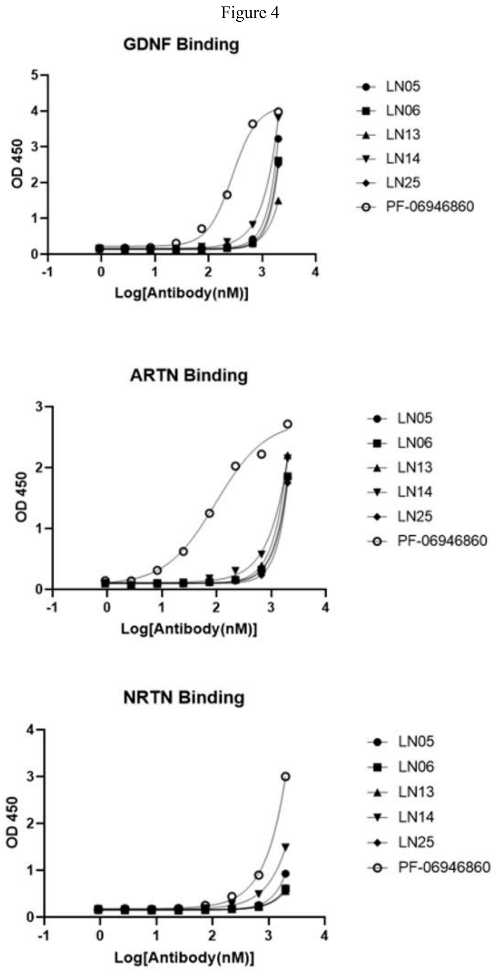

The smaller EC50 value, the stronger the binding activity and the weaker the specificity. The results are shown in Table 6 and Figure 4. LN05, LN06, LN13, LN14, and LN25 exhibited binding activity with GDNF, NRTN and ARTN only at high concentrations. PF-06946860 showed dose-dependent binding activity with GDNF and ARTN. The NRTN binding activity of PF-06946860 at the highest concentration point is higher than that of LN05, LN06, LN13, LN14 and LN25. It is inferred that LN05, LN06, LN13, LN14, and LN25 have superior specificity for GDNF, ARTN, and NRTN compared to PF-06946860.

**Table 6. Specificity of GDF 15 heavy chain single-domain antibody**

| Sample | GDNF EC50 (nM) | ARTN EC50 (nM) | NRTN EC50 (nM) |
|---|---|---|---|
| LN05 | ND | ND | ND |
| LN06 | ND | ND | ND |
| LN13 | ND | ND | ND |
| LN14 | ND | ND | ND |
| LN25 | ND | ND | ND |
| PF-06946860 | 277.5 | 97.1 | ND |
| ND: Not determined | | | |

### Example 7: Hydrophilicity and hydrophobicity study of GDF15 heavy chain single-domain antibody

The sample to be tested was diluted with mobile phase A (1.8 M ammonium sulfate + 0.1 M sodium dihydrogen phosphate solution, pH6.5) to obtain a test solution with a final concentration of 1.0M ammonium sulfate. Mobile phase A (1.8 M ammonium sulfate + 0.1 M sodium dihydrogen phosphate solution, pH6.5) and mobile phase B (0.1 M sodium dihydrogen phosphate solution, pH6.5) were used as the mobile phases. Chromatographic column: manufacturer: Sepax Technologies, model: Protemix HIC Butyl-NP5 4.6*10 mm, 5 µm; wavelength: 214 nm; flow rate: 1.0 mL/min; injection amount: 5 µg; column temperature: 25°C; sample chamber temperature: 8°C. The gradient elution method was as follows: 0~5min, 44% B; 5~15min, 44% B→100% B; 15~20min, 100% B; 20.1~25min, 44% B. Elution was performed, and the relative hydrophilicity of the target protein was determined by comparing the peak retention times of the target protein.

A shorter peak retention time indicates stronger hydrophilicity, and more hydrophilic proteins have better solubility. The results are shown in Table 7. The peak retention times of LN05, LN06, LN13, LN14, and LN25 are shorter than that of PF-06946860. It is inferred that the hydrophilicity of LN05, LN06, LN13, LN14, and LN25 is superior to that of PF-06946860.

**Table 7. Hydrophilicity and hydrophobicity of GDF15 heavy chain single-domain antibody**

| Sample | Peak retention time (min) |
|---|---|
| LN05 | 12.654 |
| LN06 | 12.741 |
| LN13 | 13.881 |
| LN14 | 11.811 |
| LN25 | 11.568 |
| PF-06946860 | 13.955 |

In the description of this specification, references to terms such as "an Example", "some Examples", "example", "a specific example", "some embodiments", or "some examples" indicate that the specific features, structures, materials, or characteristics described in combination with the embodiment or example are included in at least one embodiment or example of the present application. In this specification, the illustrative expressions of the above-mentioned terms do not necessarily refer to the same embodiment or example. Furthermore, the specific features, structures, materials, or characteristics described can be combined in any suitable manner in any one or multiple embodiments or examples. Additionally, without contradiction, a person skilled in the art can combine and integrate different embodiments or examples as well as the features of different embodiments or examples described in this specification.

Although embodiments of the present application have been shown and described above, it is understood that the above-mentioned embodiments are exemplary and should not be construed as limiting the present application. A person skilled in the art may make variations, modifications, substitutions, and alterations to the above-mentioned embodiments within the scope of the present application.

## Claims

1. An antibody or its antigen-binding fragment capable of specifically recognizing GDF15, wherein, the antibody or its antigen-binding fragment comprises:
(i) an amino acid sequence having the CDR1, CDR2, and CDR3 of a heavy chain variable region as set forth in SEQ ID NOs: 1, 2 and 4-6, wherein the CDR1, CDR2, and CDR3 are determined according to the IMGT definition, Kabat definition, Chothia definition, AbM definition, or Contact definition; or
an amino acid sequence with one or more conservative amino acid substitutions compared to (i).

2. An antibody or its antigen-binding fragment capable of specifically recognizing GDF15, wherein the antibody or its antigen-binding fragment comprises:
at least one CDR sequence selected from the following CDR sequences:
CDR sequences of the heavy chain variable region: SEQ ID NO: 7, SEQ ID NO: 8, SEQ ID NO: 9, SEQ ID NO: 10, SEQ ID NO: 11, SEQ ID NO: 12, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, and SEQ ID NO: 24; or
an amino acid sequence having at least 90% identity thereto.

3. The antibody or its antigen-binding fragment according to claim 1 or 2, wherein the antibody comprises:
CDR1, CDR2, and CDR3 sequences of the heavy chain variable region as set forth in the amino acid sequences of SEQ ID NOs: 7, 8 and 9, respectively, or CDR1, CDR2, and CDR3 sequences of the heavy chain variable region having at least 90% identity to the amino acid sequences of SEQ ID NOs: 7, 8 and 9, respectively; or
CDR1, CDR2, and CDR3 sequences of the heavy chain variable region as set forth in the amino acid sequences of SEQ ID NOs: 10, 11 and 12, respectively, or CDR1, CDR2, and CDR3 sequences of the heavy chain variable region having at least 90% identity to the amino acid sequences of SEQ ID NOs: 10 11 and 12, respectively; or
CDR1, CDR2, and CDR3 sequences of the heavy chain variable region as set forth in the amino acid sequences of SEQ ID NOs: 16, 17 and 18, respectively, or CDR1, CDR2, and CDR3 sequences of the heavy chain variable region having at least 90% identity to the amino acid sequences of SEQ ID NOs: 16, 17 and 18, respectively; or
CDR1, CDR2, and CDR3 sequences of the heavy chain variable region as set forth in the amino acid sequences of SEQ ID NOs: 19, 20 and 21, respectively, or CDR1, CDR2, and CDR3 sequences of the heavy chain variable region having at least 90% identity to the amino acid sequences of SEQ ID NOs: 19, 20 and 21, respectively; or
CDR1, CDR2, and CDR3 sequences of the heavy chain variable region as set forth in the amino acid sequences of SEQ ID NOs: 22, 23, and 24, respectively, or CDR1, CDR2, and CDR3 sequences of the heavy chain variable region having at least 90% identity to the amino acid sequences of SEQ ID NOs: 22, 23, and 24, respectively.

4. The antibody or its antigen-binding fragment according to any of claims 1 to 3, wherein the antibody comprises a heavy chain framework region sequence, and at least a portion of the heavy chain framework region sequence is derived from at least one of a camelid antibody, an alpaca antibody, a murine antibody, a primate antibody, or a mutant thereof.

5. The antibody or its antigen-binding fragment according to claim 4, wherein the heavy chain framework region sequence is derived from an alpaca antibody.

6. The antibody or its antigen-binding fragment according to any of claims 1 to 5, wherein the antibody has a heavy chain variable region with an amino acid sequence as set forth in any of SEQ ID NOs: 1, 2 and 4-6.

7. The antibody or its antigen-binding fragment according to any of claims 1 to 6, wherein the antibody comprises a heavy chain constant region sequence, and at least a portion of the heavy chain constant region is derived from at least one of a murine antibody, a primate antibody, or a mutant thereof.

8. The antibody or its antigen-binding fragment according to claim 7, wherein the heavy chain constant region of the antibody is derived from a human IgG antibody or a mutant thereof.

9. The antibody or its antigen-binding fragment according to claim 7 or 8, wherein the heavy chain constant region of the antibody is derived from human IgG1.

10. The antibody or its antigen-binding fragment according to any of claims 7 to 9, wherein the sequence of Fc in the heavy chain constant region of the antibody is as set forth in SEQ ID NO:25.

11. The antibody or its antigen-binding fragment according to any of claims 1 to 10, wherein the antibody has a heavy chain with an amino acid sequence as set forth in any of SEQ ID NOs: 26-30.

12. The antibody or its antigen-binding fragment according to any of claims 1 to 6, wherein the antibody is a small molecule antibody.

13. The antibody or its antigen-binding fragment according to claim 12, wherein the small molecule antibody comprises at least one of a single-domain antibody, a Fab antibody, an Fv antibody, and a minimal recognition unit.

14. The antibody or its antigen-binding fragment according to any of claims 1 to 6, wherein the antibody is a single-domain antibody.

15. A nucleic acid molecule, wherein the nucleic acid molecule encodes the antibody or its antigen-binding fragment according to any of claims 1-14.

16. An expression vector, wherein the expression vector carries the nucleic acid molecule according to claim 15.

17. The expression vector according to claim 16, wherein the expression vector is a prokaryotic expression vector, a eukaryotic expression vector, or a virus.

18. A recombinant cell, wherein the recombinant cell carries the nucleic acid molecule according to claim 15, or the expression vector according to claim 16 or 17, or expresses the antibody or its antigen-binding fragment according to any of claims 1 to 14.

19. The recombinant cell according to claim 18, wherein the recombinant cell is obtained by introducing the expression vector according to claim 16 or 17 into a host cell.

20. A pharmaceutical composition, wherein the pharmaceutical composition comprises at least one of the following:
the antibody or its antigen-binding fragment according to any of claims 1-14;
the nucleic acid molecule according to claim 15;
the expression vector according to claim 16 or 17; or
the recombinant cell according to claim 18 or 19.

21. Use of the antibody or its antigen-binding fragment according to any of claims 1 to 14, the nucleic acid molecule according to claim 15, the expression vector according to claim 16 or 17, the recombinant cell according to claim 18 or 19, or the pharmaceutical composition according to claim 20 in the preparation of a drug for diagnosing, treating, or preventing a GDF15-related disease.

22. The use according to claim 21, wherein the GDF15-related disease includes a cardiovascular disease, a kidney disease, obesity, diabetes, a tumor, and cachexia;
optionally, the cardiovascular disease includes myocardial hypertrophy, heart failure, atherosclerosis, and endothelial dysfunction;
optionally, the tumor is a solid tumor, including pancreatic cancer, intestinal cancer, ovarian cancer, prostate cancer, primary liver cancer, lung cancer, colorectal cancer, gastric cancer, hepatocellular carcinoma, non-small cell lung cancer, urothelial/renal cell carcinoma, and breast cancer.

23. A kit for detecting GDF15, wherein the kit comprises the antibody or its antigen-binding fragment according to any of claims 1 to 14.

24. Use of the antibody or its antigen-binding fragment according to any of claims 1 to 14, the nucleic acid molecule according to claim 15, the expression vector according to claim 16 or 17, or the recombinant cell according to claim 18 or 19 in the preparation of a kit for detecting GDF15 or diagnosing a GDF 15-related disease.

25. A method for diagnosing, treating, or preventing a GDF15-related disease, wherein the method comprises administering to a subject at least one of the following:
the antibody or its antigen-binding fragment according to any of claims 1-14;
the nucleic acid molecule according to claim 15;
the expression vector according to claim 16 or 17;
the recombinant cell according to claim 18 or 19;
the pharmaceutical composition according to claim 20.

26. The method according to claim 25, wherein the GDF15-related disease includes a cardiovascular disease, a kidney disease, obesity, diabetes, a tumor, and cachexia;
optionally, the cardiovascular disease includes myocardial hypertrophy, heart failure, atherosclerosis, and endothelial dysfunction;
optionally, the tumor is a solid tumor, including pancreatic cancer, intestinal cancer, ovarian cancer, prostate cancer, primary liver cancer, lung cancer, colorectal cancer, gastric cancer, hepatocellular carcinoma, non-small cell lung cancer, urothelial/renal cell carcinoma, and breast cancer.

27. Use of the antibody or its antigen-binding fragment according to any of claims 1 to 14, the nucleic acid molecule according to claim 15, the expression vector according to claim 16 or 17, the recombinant cell according to claim 18 or 19, or the pharmaceutical composition according to claim 20 in the diagnosis, treatment, or prevention of a GDF15-related disease.

28. The use according to claim 27, wherein the GDF15-related disease includes a cardiovascular disease, a kidney disease, obesity, diabetes, a tumor, and cachexia;
optionally, the cardiovascular disease includes myocardial hypertrophy, heart failure, atherosclerosis, and endothelial dysfunction;
optionally, the tumor is a solid tumor, which includes pancreatic cancer, intestinal cancer, ovarian cancer, prostate cancer, primary liver cancer, lung cancer, colorectal cancer, gastric cancer, hepatocellular carcinoma, non-small cell lung cancer, urothelial/renal cell carcinoma, and breast cancer.
